# EUROPEAN PATENT APPLICATION

(11) **EP 1 314 394 A2**
(43) Date of publication of application: **28.05.2003**
(21) Application number: 02258039.3
(22) Date of filing: 21.11.2002
(51) Int. Cl.: A61B 5/055

(54) **Method and apparatus for prescribing an imaging scan and checking user input**

(30) Priority: 21.11.2001 US 683130
(71) Applicant: GE Medical Systems Global Technology Company LLC, Waukesha, Wisconsin 53188 (US)
(72) Inventor: Trevino, Scott Eugene, Waukesha, Wisconsin 53186 (US); Halleppanavar, Veena, Waukesha, Wisconsin 53186 (US); McKinnon, Graeme, Hartland, Wisconsin 53005 (US); Singh, Ranjeeta, Milwaukee, Wisconsin 53202 (US); Haworth, Robert Holden, Brookfield, Wisconsin 53005 (US)
(74) Representative: Pedder, James Cuthbert

(57) **Abstract**

The present invention is directed to a method and apparatus that hierarchically prioritizes scan parameters of a medical imaging application on a per scan basis. The present invention prioritizes the scan parameters to define a dependence relationship for each scan parameter. By defining this relationship between the scan parameters on a per scan session, the present invention can notify a user of an invalid scan parameter input. A graphical user interface is provided to facilitate entry and/or modification of scan parameter values and is likewise configured to notify the user of parameter invalidity.

## Description

The present invention relates generally to medical imaging data acquisition and graphical user interfaces and, more particularly, to a method and apparatus for prioritizing scan parameters of a medical imaging application on a per scan basis and notifying a user of scan parameter input invalidity.

With known medical imaging systems, erroneous user input for a scan parameter is presented to the user as a "pop-up" dialog window that typically indicates that a specific scan parameter value is out of range and should be modified. Some systems will automatically determine to which value the scan parameter should be changed. Some systems will display a valid range of values for all scan parameters.

These systems fail to provide any information relating to the interrelationship and interdependency between scan parameters. Typically, the change of one scan parameter value affects other parameter values displayed to the user. However, these known systems fail to explicitly notify the user that other scan parameters that are dependent upon the changed scan parameter were also changed to a new value. Typically, the user is not made aware that the dependent values have changed unless the value for one of the scan parameters is modified to a value that is outside a minimum/maximum range.

Furthermore, these known systems establish the scan parameter dependencies to be constant across multiple scan applications which limits the user's ability to prescribe the imaging scan to fit the particular requirements of the scan session.

Therefore, it would be desirable to design a method and apparatus for prioritizing scan parameters for an imaging application on a per scan session basis and further design the method and apparatus to notify a user of invalid scan parameter inputs.

The present invention is directed to a method and apparatus that hierarchically prioritizes scan parameters of a medical imaging application on a per scan basis overcoming the aforementioned drawbacks. The present invention prioritizes the scan parameters to define a dependence relationship for each scan parameter. By defining this relationship between the scan parameters on a per scan session, the present invention can notify a user of an invalid scan parameter input. A graphical user interface is provided to facilitate entry and/or modification of scan parameter values and is likewise configured to notify the user of parameter invalidity.

Therefore, in accordance with one aspect of the present invention, a method of guiding prescription of a medical imaging scan is provided. The method includes launching an imaging application and determining a plurality of scan parameters for the imaging application. The method further includes comparing a scan parameter input to a reference value and determining a state of validity of a number of remaining scan parameters. The method also includes notifying a user of the state of the validity.

In accordance with another aspect of the present invention, a method of prescribing imaging data acquisition of a subject includes determining a plurality of scan parameters specific to a scan session. The method also includes hierarchically prioritizing the plurality of scan parameters for the scan session. The method further includes re-prioritizing the plurality of scan parameters for another scan session.

In yet another aspect of the present invention, a computer program is provided and includes a set of instructions that causes a computer to display a graphical user interface (GUI) configured to assist in prescription of a medical imaging session. The instructions further cause the computer to display a window on the GUI upon receipt of a selection command wherein the window is configured to display a number modifiable scan parameters. The instructions then cause the computer to receive a command to modify a scan parameter and modify the scan parameter accordingly. The computer is then caused to determine at least one effect of modifying the scan parameter on another scan parameter and display the at least one effect on the GUI.

In a further aspect of the present invention, a medical imaging system if provided and configured to initiate an imaging application and acquire imaging data of a subject and reconstruct an image of the subject. The system includes a console configured to facilitate prescribing of a medical imaging scan. The system also includes a computer programmed to display a plurality of tabs on the console wherein each tab corresponds to a specific task of the imaging application. The computer is further programmed to detect a user selection of one of the plurality of tabs and display on the console a plurality of options associated with the selected tab. The computer is then programmed to detect user modification of at least one of the options and display on the console an indication of at least one consequence of modifying the at least one of the plurality of options.

The invention will now be described in greater detail, by way of example, with reference to the drawings, in which:-
Fig. 1 is a schematic block diagram of an MR imaging system for use with the present invention.
Fig. 2 is a representation of a graphical user interface illustrating the allocation of screen space in accordance with the present invention.
Fig. 3 is a representation of graphical user interface similar to that shown in Fig. 2 illustrating allocation of screen space in an alternate embodiment of the present invention.
Fig. 4 is a representation of a graphical user interface for setting up initial scan application parameters for one representative medical imaging application in accordance with the present invention.
Fig. 5 is a representation of a graphical user interface similar to that shown in Fig. 4 for prescribing localizers for the representative medical imaging application in accordance with the present invention.
Fig. 6 is a representation of a graphical user interface for the inputting of patient information in accordance with the present invention.
Fig. 7 is a representation of a graphical user interface for prescribing and acquiring images in accordance with the present invention.
Fig. 8 is a representation of a pop-up dialog for use with the present invention.
Fig. 9 is a representation of a graphical user interface for displaying images of a scan station.
Fig. 10 is a representation of a graphical user interface for displaying summary data for the representative medical imaging application in accordance with the present invention.
Fig. 11 is a representation of a graphical user interface for prescribing a particular medical imaging application in accordance with the present invention.
Fig. 12 is a representation of a graphical user interface for acquiring medical diagnostic image for the representative medical imaging application in accordance with the present invention.
Fig. 13 is a representation of a pop-up dialog for use with the present invention.
Fig. 14 is a representation of a graphical user interface for setting up advanced scan settings for the representative medical imaging application in accordance with the present invention.
Fig. 15 is a representation of a graphical user interface for displaying help topics for the representative medical imaging application in accordance with the present invention.
Fig. 16 is a representation of a graphical user interface for displaying protocol information for the representative medical imaging application in accordance with the present invention.
Fig. 17 is a representation of a graphical user interface for modifying scan time in accordance for the representative medical imaging application with the present invention.
Fig. 18 is a representation of a graphical user interface for modifying the resolution for the representative medical imaging application in accordance with the present invention.
Fig. 19 is a representation of a graphical user interface for modifying the contrast for the representative medical imaging application in accordance with the present invention.
Fig. 20 is a representation of a graphical user interface for modifying the signal to noise ratio for the representative medical imaging application in accordance with the present invention.
Fig. 21 is a representation of a graphical user interface for modifying slice information for the representative medical imaging application in accordance with the present invention.

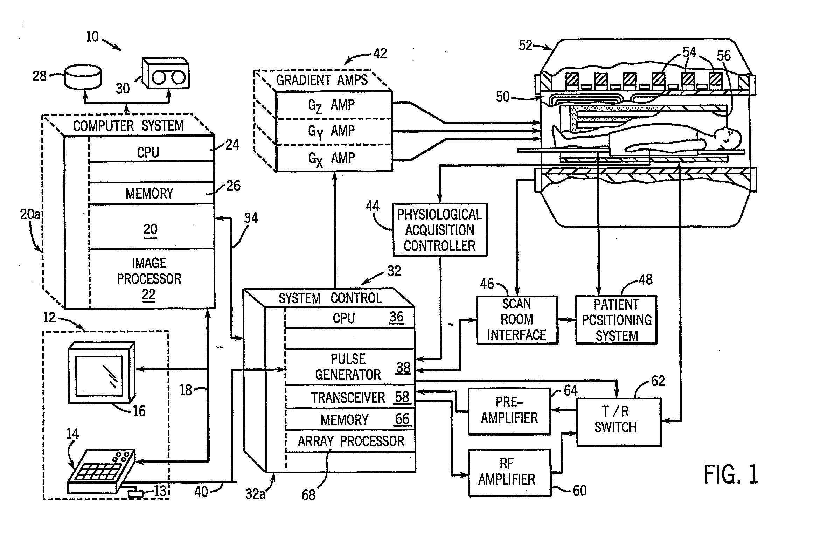

Referring to Fig. 1, the major components of a preferred magnetic resonance imaging (MRI) system 10 incorporating the present invention are shown. The operation of the system is controlled from an operator console 12 which includes a keyboard or other input device 13, a control panel 14, and a display 16. The console 12 communicates through a link 18 with a separate computer system 20 that enables an operator to control the production and display of images on the screen 16. The computer system 20 includes a number of modules which communicate with each other through a backplane 20a. These include an image processor module 22, a CPU module 24 and a memory module 26, known in the art as a frame buffer for storing image data arrays. The computer system 20 is linked to disk storage 28 and tape drive 30 for storage of image data and programs, and communicates with a separate system control 32 through a high speed serial link 34. The input device 13 can include a mouse, joystick, keyboard, track ball, touch activated screen, light wand, voice control, or any similar or equivalent input device, and may be used for interactive geometry prescription.

The system control 32 includes a set of modules connected together by a backplane 32a. These include a CPU module 36 and a pulse generator module 38 which connects to the operator console 12 through a serial link 40.

It is through link 40 that the system control 32 receives commands from the operator to indicate the scan sequence that is to be performed. The pulse generator module 38 operates the system components to carry out the desired scan sequence and produces data which indicates the timing, strength and shape of the RF pulses produced, and the timing and length of the data acquisition window. The pulse generator module 38 connects to a set of gradient amplifiers 42, to indicate the timing and shape of the gradient pulses that are produced during the scan. The pulse generator module 38 can also receive patient data from a physiological acquisition controller 44 that receives signals from a number of different sensors connected to the patient, such as ECG signals from electrodes attached to the patient. And finally, the pulse generator module 38 connects to a scan room interface circuit 46 which receives signals from various sensors associated with the condition of the patient and the magnet system. It is also through the scan room interface circuit 46 that a patient positioning system 48 receives commands to move the patient to the desired position for the scan.

The gradient waveforms produced by the pulse generator module 38 are applied to the gradient amplifier system 42 having Gₓ, G_{y}, and G_{z} amplifiers. Each gradient amplifier excites a corresponding physical gradient coil in a gradient coil assembly generally designated 50 to produce the magnetic field gradients used for spatially encoding acquired signals. The gradient coil assembly 50 forms part of a magnet assembly 52 which includes a polarizing magnet 54 and a whole-body RF coil 56. A transceiver module 58 in the system control 32 produces pulses which are amplified by an RF amplifier 60 and coupled to the RF coil 56 by a transmit/receive switch 62. The resulting signals emitted by the excited nuclei in the patient may be sensed by the same RF coil 56 and coupled through the transmit/receive switch 62 to a preamplifier 64. The amplified MR signals are demodulated, filtered, and digitized in the receiver section of the transceiver 58. The transmit/receive switch 62 is controlled by a signal from the pulse generator module 38 to electrically connect the RF amplifier 60 to the coil 56 during the transmit mode and to connect the preamplifier 64 to the coil 56 during the receive mode.

The transmit/receive switch 62 can also enable a separate RF coil (for example, a surface coil) to be used in either the transmit or receive mode.

The MR signals picked up by the RF coil 56 are digitized by the transceiver module 58 and transferred to a memory module 66 in the system control 32. A scan is complete when an array of raw k-space data has been acquired in the memory module 66. This raw k-space data is rearranged into separate k-space data arrays for each image to be reconstructed, and each of these is input to an array processor 68 which operates to Fourier transform the data into an array of image data. This image data is conveyed through the serial link 34 to the computer system 20 where it is stored in memory, such as disk storage 28. In response to commands received from the operator console 12, this image data may be archived in long term storage, such as on the tape drive 30, or it may be further processed by the image processor 22 and conveyed to the operator console 12 and presented on the display 16.

The present invention is directed to a method and apparatus of directing workflow for medical imaging experiments and sessions. The invention utilizes an hierarchial scheme to facilitate improved workflow. The workflow tool will be described with respect to a Peripheral Vascular (PV) application using MR imaging technology which is considered the "super" application because it is defined by the combination of multiple sub-applications. The teachings of this invention are not limited, however, to a PV application or MR technology. The PV application of the present invention varies from a traditional application of known MR systems. Specifically, the PV application is a combination of a 2D gradient echo application and a 3DSPGR (Three-Dimensional with Spoiled Gradient Echo Pulse Sequence) application. Therefore, the PV application GUI is a composition of the components that it defines as well as the components from other "sub" applications. The present invention includes a GUI 100 designed to dynamically adjust the layout and distribution of screen space throughout the scan. The PV application GUI can generally be thought of as a collector. As a result, nothing prohibits the "sub" applications from similarly acting as a recursive collection of any number of other application GUIs.

The present invention improves workflow by increasing the intuitiveness of the application workflow, making the application more flexible, improving usability, decreasing the number of user interactions/steps, and incorporating fault tolerance. In one preferred embodiment, the PV application may be launched by "double clicking" an icon displayed on the console 16, Fig. 1. By launching the PV application, the user may create a new exam, edit an existing protocol, and/or enter patient information.

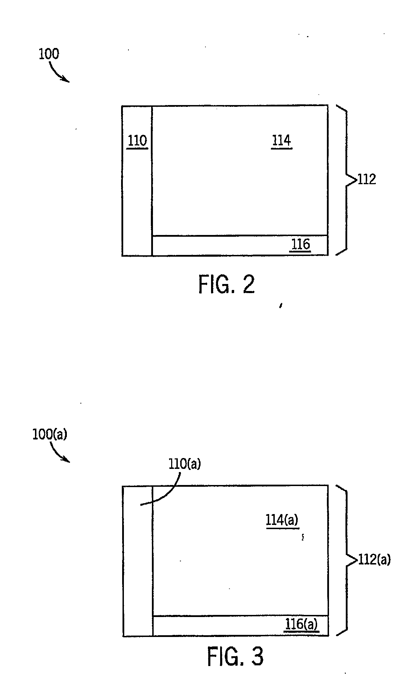

Fig. 2 is an illustration of a layout of a GUI in accordance with the present invention. GUI 100 is designed to dynamically adjust the layout and distribution of screen space throughout the scan. As illustrated, GUI 100 includes a generic control region 110 which occupies approximately 20% of the available screen space, whereas the remaining 80% of the screen space is reserved for control of a local or particular application 112. In this embodiment, the region 110 will retain 20% of the total screen space and thereby limit the space available for region 112. In this embodiment region 112 includes prescription area 114 and an operations area 116.

However, in another embodiment as shown in Fig. 3, GUI 100(a) includes space 112(a) which is distributed to include region 114(a) but region 116(a) is reserved for generic control operation. This occurs when the generic control application has the scanner resources and the control for the prescription application is simply being used to prescribe a scan session. In this embodiment, space associated with the Lx application 110(a) and 116(a) retains an additional 10 - 15% of the screen space. Therefore, the Mx application may utilize only 65 - 70% of the total screen space for conveying information.

Figs. 2 and 3 illustrate various embodiments for allocating finite screen space among several medical imaging applications. Distributing the screen space in a position similar to that shown in Figs. 2 and 3 facilitates ease of user interactions between applications. It should be noted that the present allocations described above are for illustrative purposes and are not intended to limit the scope of the invention.

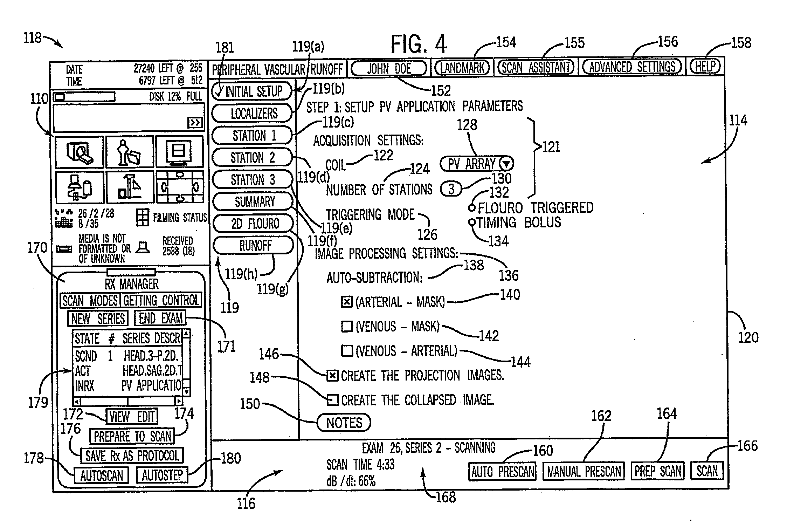

Referring now to Fig. 4, GUI 118 is shown having an initial setup window. GUI 118 is displayed when the PV application is first launched or, alternatively, when the user selects "Initial Setup" tab 119(a) of modularizing tab array 119. This view presents the user with an "Initial Setup" window 120. Window 120 allows the user to perform the initial setup for the PV application. The user may establish settings such as acquisition settings 121. Included in the acquisition settings 121 are coil 122, number of stations 124, and triggering mode 126. Corresponding to coil 122 is a drop-down menu 128 that allows a user to select a coil such as a PV array. The user may input the number of stations in field box 130 and select the triggering mode 126 by choosing fluro triggered radial button 132 or timing bolus radial button 134. If the user inputs a number stations greater than three, GUI 118 automatically updates to add additional modularizing tabs to array 119.

Array 119 not only includes "Modularizing" tab 119(a) corresponding to initial setup, but also includes a "Localizers" tab 119(b), a "Station One" tab 119(c), a "Station Two " tab 119(d), a "Station Three" tab 119(e), a "Summary" tab 119(f), a "2D Fluro" tab 119(g), and a "RunOff" tab 119(h). Modularizing tab array 119 is vertically arranged along a left side of window 120. The tabs 119(a)-(h) correspond to each prescription step of an medical imaging scan session. The nomenclendure provided for each tab is for illustrative purposes as differing medical imaging applications would utilize different tab names. The tabs are arranged vertically and, in a preferred embodiment, in order of execution. That is, tabs 119(a)-(h) are logically arranged to guide a user through prescription of the medical imaging scanning session. When a particular tab is selected by a user, the tab is highlighted in a known manner to indicate selection of the particular tab. As shown in Fig. 4, the appearance of GUI 118 is representative of that which appears upon user selection of "Initial Setup" tab 119(a).

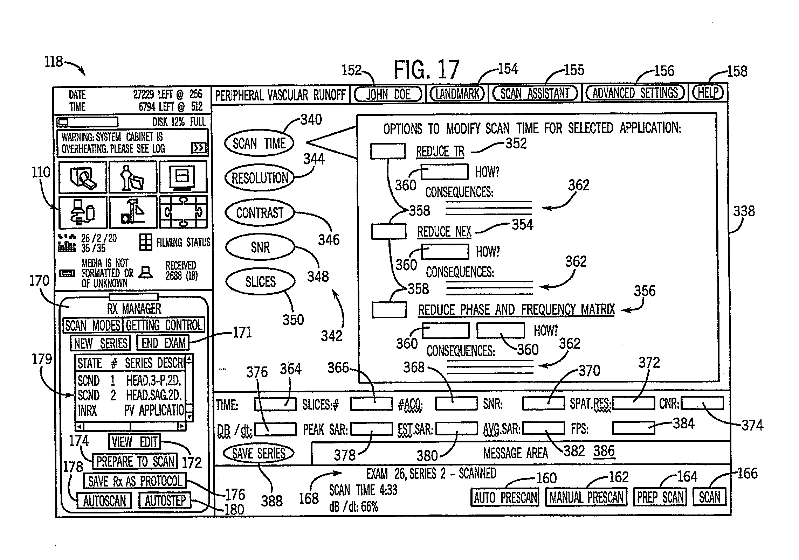

GUI 118 further facilitates user selection of image processing settings 136 such as identifying the proper auto subtraction processing 138. In a preferred embodiment, the user may select one of arterial-mask 140, venous-mask 142, or venous-arterial 144. The user may also indicate whether to create projection images by selecting check box 146 or create a collapsed image by selecting check box 148. GUI 118 further includes a "Notes" button 150 that once selected by a user will cause a GUI or window to appear for entering of notes related to the instant medical imaging scanning session or protocol. A "Patient" button 152 is also provided that upon activation by a user will display information relating to the patient. A "Landmark" button 154 as well as an "Advanced Settings" button 156 are also provided and will be discussed shortly. Selection of "Landmark" button 154 causes another window (not shown) to appear which is configured to facilitate proper positioning of the scan subject. If the user has any questions or needs assistance relating to the prescription steps, the user may select "Help" button 158 to display various topics to assist the user with prescribing the imaging scan. "Scan Assistant" button 155 will be discussed with reference to Figs. 17-21.

As indicated previously, GUI 118 includes a prescription region 114 and generic control regions 110, 116. Region 116 includes an "Auto Pre-Scan" tab 160, a "Manual Pre-Scan" tab 162, a "Prep Scan" tab 164, and a "Scan" tab 166. User selection of these tabs 160-166 varies depending upon the particular application. Region 116 also includes status identifiers 168 that display the current scan time, completion status, and activation status.

Region 110 includes an Rx manager interface 170 that displays various information regarding the particular prescription. The Rx manager 170 includes a "View/Edit" tab 172, a "Prepare To Scan" tab 174, a "Save Rx As Protocol" tab 176, an "Auto Scan" tab 178, and an "Auto Step" tab 180. Tabs 172-180 will display upon user selection thereof a corresponding window to facilitate user completion of the selected task or activity. A number of additionally status indicators and tabs are also provided in region 110 to provide information to the user as to the status of the scan session.

In a preferred embodiment, the user will make changes to the PV application settings when defining a new protocol. That is, a user may make selections in window 120 of GUI 118 and throughout other portions of the application, such as an "Advanced Settings" window (to be discussed shortly), and then save the settings as a new protocol. As a result, all subsequent executions of this PV application could utilize the created protocol and the user would typically only review the settings in the "Initial Setup" page and then click the next tab, the "Localizers" tab 119(b), to begin the acquisition of data. When the user has entered all of the data for a particular tab, a check 181 will appear as a label to indicate that the necessary steps have been achieved.

Still referring to Fig. 4, there are three stations for this application as indicated in the "Number of Stations" text field 130. This is important because the number of stations determines the number of corresponding steps/tabs 119 for this application. Specifically, there is one tab per station for the acquisition of the 3D volume mask images and there is one localizer image set acquired per station. For example, if there were only two stations defined there would be one fewer tab (i.e. "Station 3" tab 119(e) would not be necessary), only two localizers listed under the "Localizers" tab 119(b), and only two stations for arterial and venous images. If the user entered six stations on the "Initial Setup" page 118, the number of tabs 119 would update to add three more (i.e. "Station 4", Station 5", and "Station 6"), there would be six localizers under the "Localizers" tab 119(b), and six stations for arterial and venous images.

The "Arterial-Mask" option 140 specifies that after acquisition of the arterial run images a set of subtracted images should be automatically generated using the masks. It should be noted that the auto-subtraction option 138 should be an improvement over existing systems as it automates and simplifies this application.

Workflow within this application works in the following way. A user navigates an application through a series of steps as conveyed by the tabs 119 on the left side of the screen 114. There is a one-to-one relationship between the number of tabs 119 and the number of steps in the PV application. Therefore, in this embodiment, the PV application has eight steps corresponding to the number of tabs 119. Preferably, the user moves through these tabs 119 from top to bottom. This is expected to be the preferred manner of completing this application, however, the user may complete the steps in any order. As all the tasks with each tab 119 are completed (i.e. the "Localizer" tab 119(b) is only considered complete when the task of acquiring the localizers is completed) each tab 119 displays a checkmark icon 181. This icon will indicate to the user that the step has been successfully completed. If a step has not been completed, partially or not at all, the tab will not have a check. Also, all seven steps prior to the "RunOff" step (i.e. the last step) must have been successfully completed in order to acquire the arterial and venous runs. That is, the PV application requires that all steps prior to the final step of arterial and venous acquisition be performed. The user will be notified of this requirement, if they try to acquire the "runs" without completing all prior steps, via the "Scan" button 166 being disabled and a message being displayed in the "Application Message" area 116.

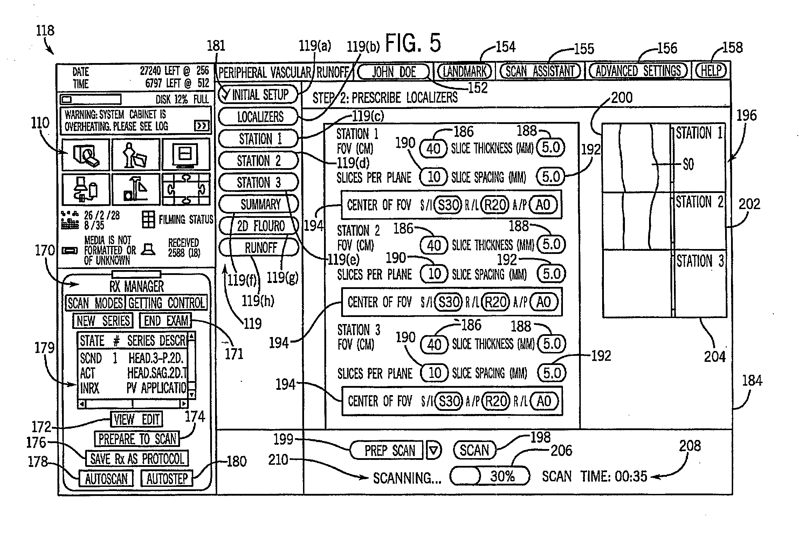

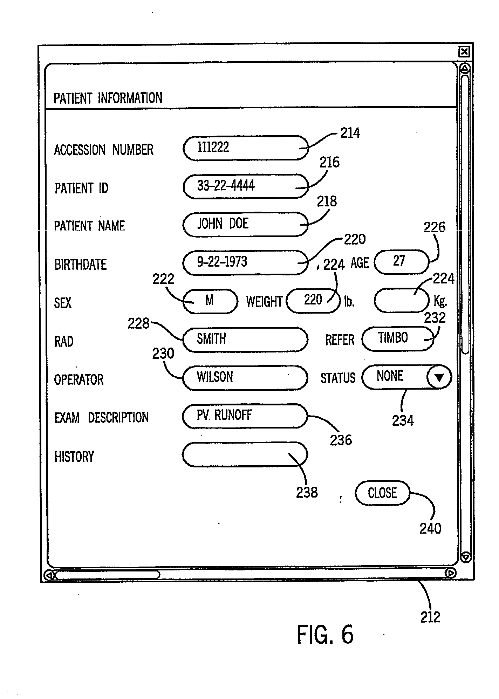

Referring now to Fig. 5, a representation of GUI 118 upon user selection of "Localizers" tab 119(b) is shown. Window 184 appears within GUI 118 and allows the user to review and/or change the scan parameters for each of the station localizers (as defined in the "Initial Setup" mentioned earlier). Fig. 5 is an illustration of how the user may multi-task effectively by "prescribing ahead" a local application while the system is busy scanning another generic series. The user may view "Patient Information" by clicking button 152 at the top of the screen in the "Global Information Access" area that contains the name and ID of the patient. A pop-up dialog will then be displayed on top of the PV application GUI 118 similar to that shown in Fig. 6 (which will be described below).

Window 184 allows the user to review and/or change the scan parameters for each station. The user may adjust the FOV 186, slice thickness 188, slices per frame 190, and slice spacing 192 for each station. The user may also review and/or edit scan parameters relating to the center of the FOV 194.

Once the user inspects and verifies the scan parameters presented, the user may select "Prepare to Scan" button 198 to initiate a resource switch to transfer the scanning resources and download. The user can then select "Scan" to initiate a scan for the localizer application and perform any necessary Prescan operations and then scan the localizers. The resource switch is a very important difference between the present system and other known systems. In the present invention, one must consider the consequences of the first selection of a scanning operation. This will cause a scanning resource switch, whether it is the first selection of a scan operation in the localizer application when the scanner is "owned" by the global application, or vice versa. Therefore, when a user selects scan, the first thing that occurs is a resource switch.

A "Humanoid" 196 is displayed in a right portion of window 184. When the "Scan" button 198 is selected, all three localizers are automatically scanned and images are displayed in the "Humanoid". This is an important step in improving the user workflow by automating redundant steps and streamlining how the user moves through this system. In a preferred embodiment, one cannot scan localizers in any other fashion. If there are more or less stations defined, as part of the initial setup, then there will be fewer or more localizers to be acquired. In either case, the localizer acquisition will be done automatically.

After selecting "Scan" button 198, the GUI 118 will set forth the progress being made towards completion of the resource switch and scan in one of three ways.

First, the "Humanoid" 196 displayed to the immediate right of the localizer scan parameters window 184 will display localizers from each station as they are being acquired. That is, when the first localizer image from the first station (most superior in this case) is acquired the middle sagittal image 200 will be displayed in the top viewer of the "Humanoid" 196. Each subsequent image 202, 204 acquired for that station is also displayed. The "Humanoid" 196 provides the capability for the user to scroll through the images 200-204. However, in one embodiment, the images displayed will only be sagittal images. As the system finishes acquiring the localizer from one station and then begins acquisition of a localizer at another station, the "Humanoid" 196 updates as necessary until the scanning completes.

The second way in which the user is made aware that the global application system is scanning is via progress bars 206 and a timer 208, both of which indicate the progress towards the completion of the resource switch and localizer acquisition. Another bar (not shown) shows progress towards the completion of the resource switch on the scanner. Bar 206 indicates the percentage of the task completed based on images acquired versus total images. The "resource switch" progress bar will be displayed first and will be replaced by the "image acquisition" progress bar immediately after it completes. Timer 208 shows the count down of time for the image acquisitions. Timer 208 will be displayed when the "Scan" button 196 is selected, but will not begin counting down until the scanner actually begins the scan.

The final way in which the user is made aware that the global application system is scanning is via the desktop icon displaying the word "Scanning" 210, the scan operation buttons being disabled, and, in most experiments, the user can hear the scanner as it is scanning.

Referring now to Fig. 6, "Patient Information" window 212 appears upon user selection of patient tab 152, Fig. 4. Window 212 allows the user to view an accession number 214, a patient ID 216, name 218, birth date 220, sex 222, weight 224, age 226, radiologist 228, operator 230, reference 232, status 234, exam description 236, and history 238. A "close" button 240 is also provided to allow the user to close window 212.

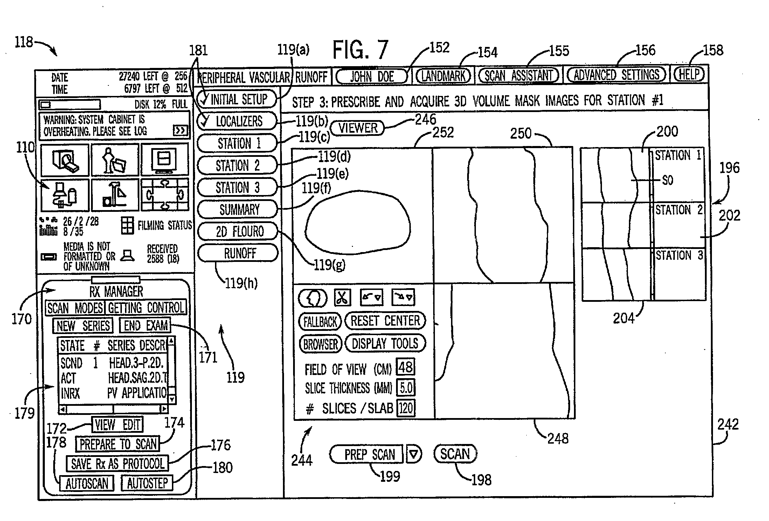

Referring to Fig. 7, once the user has acquired the localizers for the three specified stations, the user may select the next step, "Station 1" tab 119(c), in order to display window 242 to prescribe and acquire the 3D mask images for the first station. The user may also proceed to the next step before acquisition of images. In this embodiment, the user cannot perform any further interactions associated with this step as the required localizer images have not been acquired. Alternatively, the user may select scan and move to the next step while the image acquisitions are occurring. In this embodiment, the user can begin the next step once the first localizer is acquired. Window 242 contains the same "Humanoid" 196 in the same location as in Fig. 5. However, instead of the localizer imaging parameters for each localizer being presented, there is a 3-pane GRx tool 244. Directly above the GRx tool 244 is a toggle button 246 that allows the user to move between viewing the acquired 3D mask images 248-252 and interacting with the 3-plane GRx tool 244. Below the GRx tool 244 is the "Prep Scan" combination button 199 and the "Scan" button 198 as shown in Fig. 5. These two buttons will not become active until after the user places the prescription on the image and no other application is scanning.

Once the 3D volume has been placed on the localizer images the user may ineract with the 3D volume by dragging and rotating the 3D volume. Also, the user may use the tools located in GRx 244.

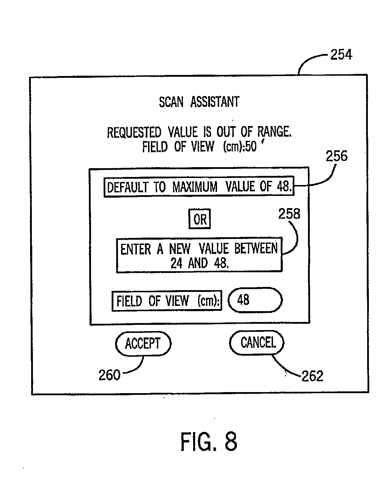

Referring to Fig. 8, most medical imaging applications employ policies for its scan and application parameters that prevent the user from entering invalid prescriptions. One tool that enforces these policies is referred to as " Scan

Assistant" window 254. In the PV application, the policy will be to "popup" a dialog 254 whenever a user enters parameters that are invalid. This dialog 254 will indicate to the user the error and force selection of another valid value. The user may choose between a default value 256 the system chooses, which is the next closest value to the invalid entry, or may enter another valid value 258. This tool 254 will prevent the medical imaging application from being in an invalid state. User may accept the changes by selecting "accept" tab 260 or cancel the change by selecting tab 262. An alternate "Scan Assistant" tool will be described with respect to Figs. 17-21.

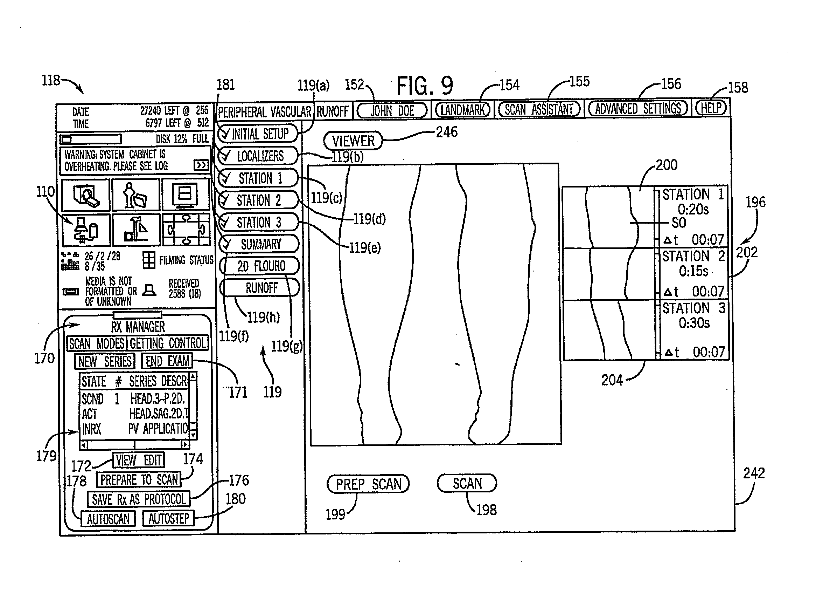

Referring again to Fig. 7, to acquire the 3D mask images for this station, the user would select "Scan" button 198. As described earlier, the "image acquisition" progress bar 206 and timer 208 are displayed while acquiring the images. Also, once the "Scan" button 198 is selected, the area of the screen occupied by the GRx tool 244 is replaced with an image viewer, Fig. 9. Referring now to Fig. 9, the image viewer 263 associated with "Station 1" button 119(c) is displayed can be used to scroll through the acquired images as well as performing basic image operations such as window level and pan/zoom. In addition, the "Humanoid" 196 displays the 3D volume that was prescribed on the associated localizer and a "GRx/Viewer" toggle button 246 becomes active.

The "Humanoid" 196 also enables the viewer to display the localizer images selected to gain focus and also allows for the images in these viewers to be scrolled, pan/zoomed, and window leveled. The "Humanoid" 196 enables viewers to be selected which causes the PV application to switch to the associated prescription. For example, if the user "double-clicks" the third viewer in the "Humanoid" (i.e. station 3), the window associated with "Station 3" tab 199(e) will become selected and the user can move forward with this step. Further, "Humanoid" 196 displays information such as the iso-center, station number, station acquisition time, and the time for table motion. Because there are three stations defined there are three 3D masks to be prescribed and acquired.

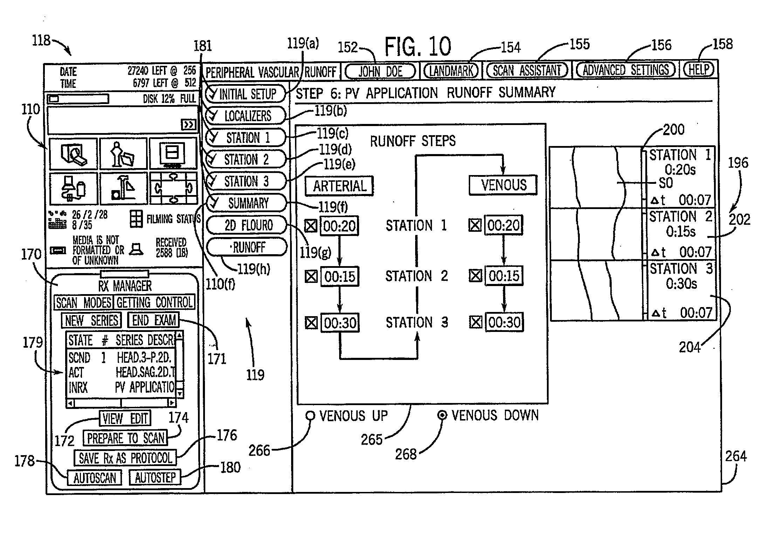

Referring now to Fig. 10, after all mask image sets for each station have been acquired, the user may proceed to the "Summary" tab 119(f). The purpose of "Summary" window 264 is to present the user with the option of reviewing the acquisition order, time to acquire the arterial and venous images, and to "skip" acquisition of any arterial or venous phase or to change number of phases. All of this is accomplished via the information panel 265 displayed to the left of the "Humanoid" 196.

Window 264 clearly illustrates to the user everything that is scheduled to occur during the acquisition of the arterial and venous images. Things illustrated include:

Two columns indicating the arterial and venous acquisitions through the use of colored labels (i.e. red for arterial, blue for venous).

Colored labels contain the scan time for each series.

Check boxes next to the boxes allow the user to select or skip the acquisition. Therefore, in order to skip any step, the user only has to uncheck the check box associated with the particular acquisition.

Panel clearly shows the start of the acquisition as well as the total time listed for the acquisition. This number will dynamically update based on order selected and what is and is not being acquired.

In addition to the panel 265, there are also two buttons 266, 268 that the user can choose from in order to define the order of arterial and venous acquisition. One selection, "Venous Up" 266 acquires the arterial images superior to inferior and then the venous images inferior to superior thus reducing table movement. The second option is "Venous Down" 268 which acquires both the arterial and venous images superior to inferior. In one embodiment, "Venous Down" 268 is selected by default.

In addition to all that can take place during the "Summary" step, the present invention allows the user to re-acquire the 3D mask images for a particular station. Since the user may change the prescription for the 3D masks for station two and then re-acquire the images, the user need follow the same steps mentioned above when they first prescribed and acquired the 3D masks for station two. That is, "Station 2 " tab 119(d) is selected and the GRx tool is used to fix the prescription. The user then presses the "Scan" button. Reacquisition of mask images for station 2 does not affect the previously acquired data for the other stations. Once this is completed, the user selects the "Summary" tab once again to again review a summary of the data acquisition.

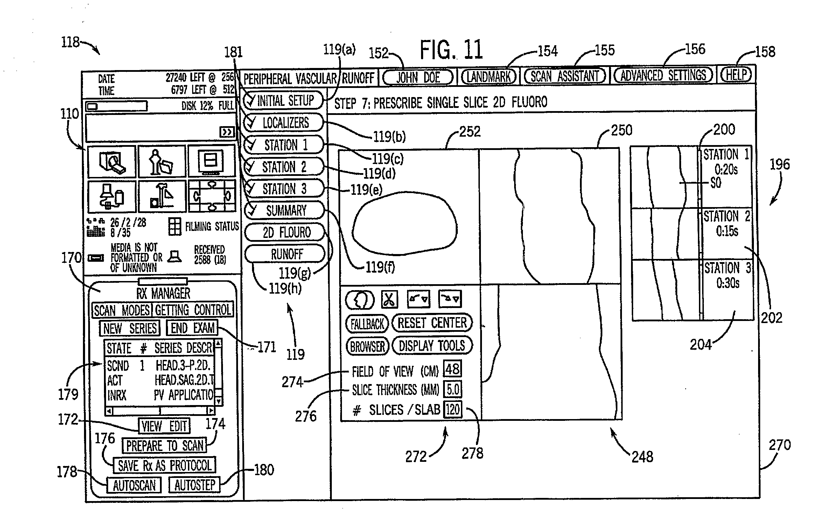

Referring to Fig. 11, the present invention allows for prescribing of a fluoroscopy by selecting modularizing tab 119(g) from GUI 118. Upon selection of tab 119(g), window 270 is displayed. Window 270 includes a GRx tool 272 for 2D prescription that enables the user to input various fluoroscopy parameters such as FOV 274, slice thickness 276, and number of slices per slab 278. "Humanoid" 196 remains displayed in a right portion of the screen as well as localizer images 248-252.

After prescribing the Fluoro acquisition, the user may then select "RunOff" tab 119(h) to complete the final step in the imaging application.

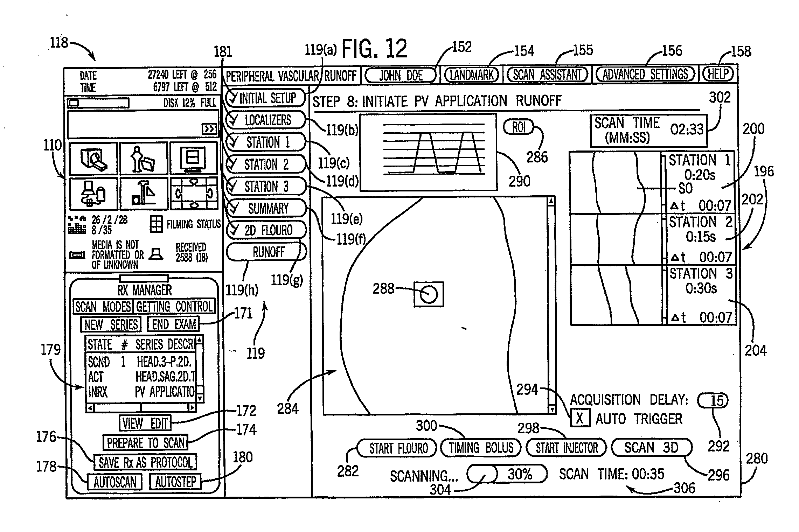

Referring to Fig. 12, window 280 appears when tab 119(h) is selected. From window 280, the user can acquire arterial and venous images in one of two ways. First, the user may use a real-time fluoroscopy technique to acquire the images. To acquire the images the user will begin by pressing the "Start Fluoro" button 282, which will cause the viewer on this page to present the user with a real-time image 284 of the location that was prescribed and the "Start Fluoro" button will change its label to read "Pause Fluoro". At this point the user could do one, none or both of the following:
A. Select the "ROI" button 286 and draw a Region of Interest (ROI) 288 over the area of interest on the image in the viewer 284. This step is not required as the user could also visually detect bolus arrival. In this particular case, an ROI is used and as soon as it is placed on the image, the 290 in the top of window 280 updates with pixel intensity information.
B. Enter a time manually into the "Acquisition delay" text field 292. This can only be done if the "Auto Trigger" 294 is selected. In this case, the user leaves text field 292 at zero which tells the system that the user must manually press the "Go 3D" or "Scan" button 296 to initiate a scan.

After implementing the Fluoroscopy, the user may start the injector by pressing the "Start injector" button 298 which will essentially begin the injection of the contrast agent. If the "Acquisition Delay" 292 had a value greater than zero, the viewer would start a timer and would auto-scan when it reaches the same value displayed in the "Acquisition Delay" text field 292 if "Auto Trigger" 294 was selected. The user may watch the image 284 in the viewer as well as the graph 290 in order to detect the arrival of the contrast. Once the contrast is detected, it is time to begin the scan. The user may give any necessary instructions to the patient (i.e. hold breath) and press the "Scan" button 296, which will cause the sequence of arterial and venous image acquisitions to occur as prescribed in the "Summary" step. As these images are being acquired, they will be automatically displayed in the viewer. The user may scroll, pan/zoom, and window level these images.

A second way in which the user may acquire arterial and venous images is through the use of a timing bolus. To do this, the user must first prescribe the location for the fluoro image. The user may then start the fluoro acquisition by pressing the "Start Fluoro" button 282. As the fluoro acquisition is occurring in real-time, the user may prepare themselves and the patient and then press the "Timing Bolus" button 300. This will cause a few things to occur. First, button 300 will change to read "Mark Time" and still be active. Second, the image will display a timer 302 that is incrementing in seconds from the time the "Timing Bolus" button 300 is pressed and will not stop until the "Mark

Time" button 300 is pressed. The final change from pressing the "Timing Bolus" button 300 is that the injector will inject a small amount of bolus into the patient, which the user will use to time the arrival of contrast into the fluoro image.

After the "Timing Bolus" button 300 is pressed, the user will watch the image 284, and possibly the graph 290, for the contrast to arrive. When the contrast is detected, the user presses the "Mark Time" button 300. This action will cause timer 302 on the image to stop incrementing. Further, a "Time to Start" text field (not shown) will become active with the same value as the timer on the image. Next, the user may decide to change the value of the "Time to Start" text field by simply highlighting the field and entering in a new value, or leave it as is. (Note: Throughout this process, the fluoro acquisition continues to occur.) Now the user may acquire the arterial and venous run images.

When "Start Injector" button 298 is pressed, the full amount of contrast agent is injected into the patient and the value in the text field and the timer on the image will begin counting down therefore functioning as a visual queue/reminder to the user. If the auto trigger 294 is selected, the value in the text field and on the image reaches zero and scanner automatically begins acquiring the arterial and venous images. The user may manually press the "Go 3D" button 296 before the timer in the viewer reaches the value displayed in the "Time to Start" text field, but not after. If the auto trigger is not selected, the value in the text field and on the image only serves as a "guide" to the user that they should manually select the "Go 3D" button 296 when it reaches a value of zero. However, when the value does equal the "Time to Start" text field, nothing happens. Therefore, it is up to the user in this case to initiate the scan. They may do it before, after, or when the times equal. When the scan is initiated, a scanning timing bar 304 is displayed as well as a scan time timer 306.

After the user has completed the acquisition of the arterial and venous images the user may save this particular instance of the PV application as a protocol that may be implemented at a later date without reentering each parameter. This allows for build-up of a protocol database that may be accessed in the future. To save the protocol, the user selects the "Save Rx as Protocol" button 176 inside the Rx Manager 170 on left side of the GUI.

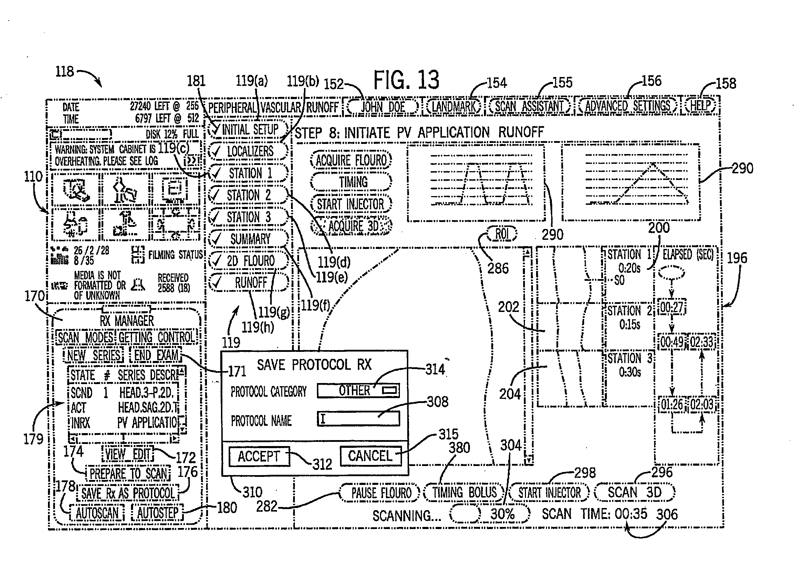

Next and referring to Fig. 13, the user may enter the identifying name for this protocol in text field 308 of the "Save Protocol Rx" dialog 310 that pops up and select the "Accept" button 312. The user may also identify a protocol category using drill down menu 314. To cancel "saving" of the protocol the user may select button 315.

After this application is saved as a protocol, the user may want to close the exam as all series have been scanned. In order to end the exam, the user selects the "End Exam" button 171 on the left side of GUI 118. This will cause the current contents of the scan window to be closed.

Referring again to Fig. 4, the present invention allows for viewing and/or editing a screen series by selecting the "View Edit" button 172, or by double clicking a desired series 179. Either of these actions will cause the currently displayed window (immediately to the right of the Rx Manager) to be hidden, and the window associated with the selected series to be shown.

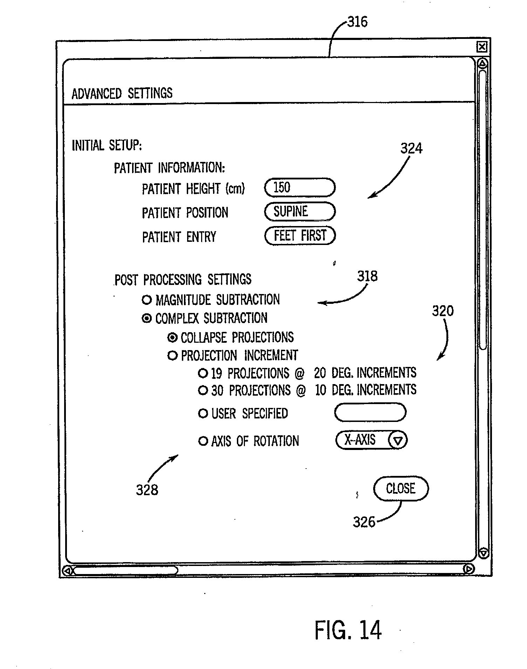

Referring to Fig. 14, the present invention includes an "Advanced Settings" window 316 which allows the user access to all parameters, features, and tools associated with a particular application for viewing and/or editing. For example, window 316 allows the user to access parameters associated with image subtraction 318, image projections 320, as well as all scan and application parameters 322 that are not presented to the user throughout the steps of the application. The user may also view/edit advanced settings regarding patient information 324.

Additionally, when the user launches the "Advanced Settings" window, the presentation within the dialog window will contain the parameters and advanced settings for the currently selected step in the application. This will be referred to as "context sensitive" behavior. For example, if the user has the "Initial Setup" window selected when the "Advanced Settings" button is clicked, the window that displays will be set to the parameters and advanced settings for the initial setup. Also, this dialog will contain the parameters and advanced settings for all components of the application, which can be reached via the scroll bar on the right-hand side of the dialog window. Note that the parameters and advanced settings are organized and listed in the dialog window in the same order that they appear in the application (i.e. "Initial Setup", "Localizers", ..., "RunOff"). Once the user completes viewing/editing, window 316 may be closed by selecting button 326.

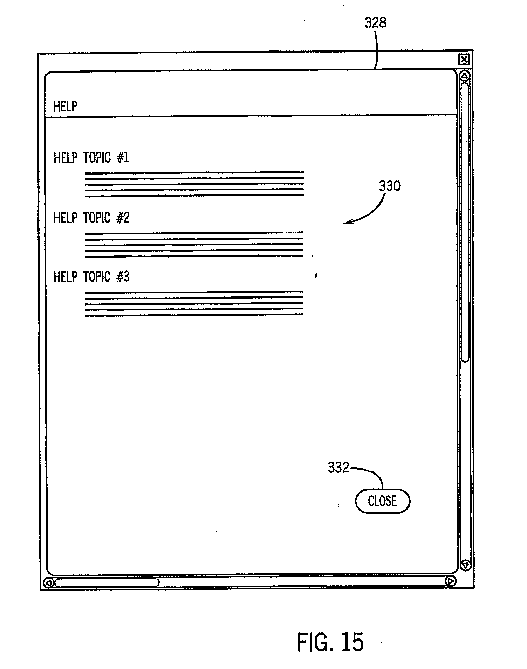

Referring to Fig. 15, a "Help" window 328 appears upon selection of "Help" button 158, Fig. 4. A number of help topics 330 may be listed to help the user clarify any issue. The help topics 330 may be application specific or specific to the activities of a particular tab 119(a-h).

Much like the "Advanced Settings" window, Fig. 13, the "Help" dialog is context sensitive. So, in this case when the dialog comes up the first choices presented to the user should relate directly to the currently selected step. Therefore, if the "Initial Setup" step was selected, the options in the "Help" window 328 should include projection and collapse images amongst other topics. Also, window 328 will allow the searching of all topics contained in the help system. The purpose of the help system, will be to answer user questions regarding how to complete an application, medical imaging physics questions, and serve as a place holder for user notes about a particular topic or application. The user may select close button 332 to close window 328.

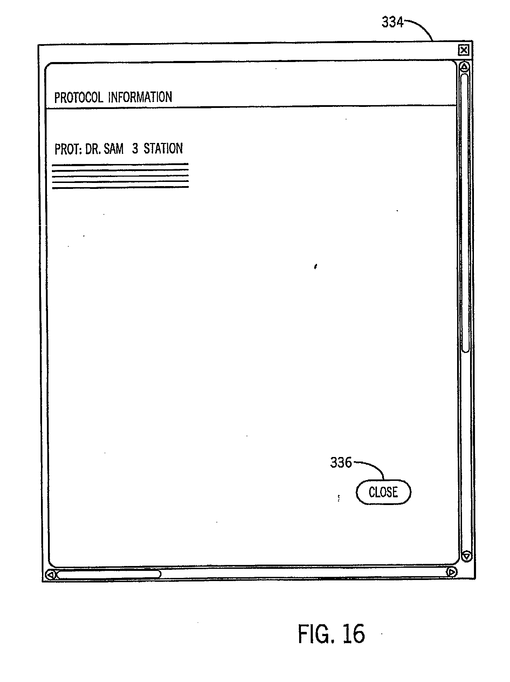

Referring to Fig. 16, a protocol window 334 may be viewed which displays the contents that are not "context sensitive". That is, the protocol information window 334 will always contain the same options for each application. All that will change between instances of the application are the values and settings for these options. Also, in one embodiment, these options can only be viewed in window 334 as they are not editable. After viewing the protocol information, the user may close window 334 by selecting close button 336.

As discussed above, the present invention includes an "Advanced Settings" window whose context is adaptive to display those parameters and settings associated with a particular tab. These settings allow access to all possible application parameters and features for users that have special needs. For example, the "Localizer" tab in the PV application only displays a few scan parameters for each station. These options have been determined to be the most important, but some users may want access to other options. If so, the user need only select the "Advanced Settings" button and a page will be presented with all available options and features of the specific imaging application. The information that will be displayed to the user when the "Advanced Settings" button is pressed will depend on the currently selected step in the application. Like the "Help" window, the "Advanced Settings" window will be context sensitive in that it will display the parameters and advanced settings for the particular step in the application that is selected when the "Advanced Settings" is pressed. However, the user can still access any of the other parameters and advanced settings available for other steps in the application. The Advanced Settings for each modularizing tab are set forth below:

### 1. Initial Setup:

Patient Height
Patient Position
Patient Entry
Magnitude Subtraction
Complex Subtraction
   Collapse Projections
   Projection Increment
      19 projections @ 20 deg. Increments
      38 projections @ 10 deg. Increments
      User Specified
      Axis of Rotation

### Localizers:

FOV
Slice Thickness
Spacing
Frequency
Phase
NEX (%)
Phase FOV
Auto Center Frequency
Autoshim
Contrast
Coverage; center of FOV (R/L, A/P, S/I)
Number of slices per plane
Scan controls (scan, prescan, manual prescan, auto prescan)
Different number of images per 3-plane

### 3. 3D Rx:

Plane
Mode
TE
Flip Angle
Bandwidth
FOV
Slice Thickness
Locs per slab / # of slices
Frequency
Phase
NEX
Phase FOV
Frequency direction
Auto center frequency
# of slabs

It uses the following options:
Variable bandwidth
ZIP2
ZIP512
CV10 → Special (on/off)
CV12 → Elliptic Centric (on/off)
Referse elliptic centric

### 4. Summary:

None

### 5. Fluoro Rx:

Plane
Mode
TE/TI
Tr
Flip Angle
Bandwidth
FOV
Slice Thickness
Matrix Frequency
Matrix Phase/PFOV
NEX
Frequency Direction
Auto Center Frequency

As indicated previously, the present invention utilizes a "Humanoid" configured to function as a visual tool that allows the user to interact with and navigate the application, gather data about the exam, and view images. The "Humanoid" displays localizer images for each station and allows access to a station's GRx viewer by "double-clicking" on the corresponding image. Further, the images will display prescription overlap from one view image to the another. "Double-clicking" an image in the "Humanoid" will immediately take the user to the step corresponding to that station's GRx. For example, selecting the middle viewer on the "Humanoid" will cause the current window to change to the window that would appear as if the "Station 2" tab had been selected. The station label will change slightly when the user is prescribing that station to indicate to which is the active station. The scan times displayed on the "Humanoid" will be updated dynamically based on changes. A user can scroll through the selected images in a viewer. A user can window/level the selected images in a viewer. A user can select and view different localizer planes on the "Humanoid" as well.

The present invention allows for messages to be displayed to a user. The error messages may be separated into two categories: application level messages and system/safety messages. System and safety level messages may be displayed in the upper left hand side of the GUI 118, Fig. 4. There are a couple of ways in which application level messages will be presented to the user. First, text messages may be placed within the applications panel underneath the tabbed pane and above the "Scan Ops" area of the screen. Another way in which these messages may be presented is through pop-up displays to the user. In the former case, the messages will typically be informational. The messages in the latter case will be due to erroneous user input into scan parameter fields.

In a further embodiment, the present invention includes a series of graphical windows that for the purposes of this application will be collectively referred to as a "Scan Assistant". In known systems, the mechanism for preventing erroneous input of scan parameters by a user is to present to the user change in scan parameter label colars indicates a specific scan parameter value is out of range and needs to be changed to a suggested value. While the user is shown a valid range of the value read scan parameter, these systems fail to provide any information to indicate that scan parameters are inter-related and can depend on one another. If the value of one scan parameter is changed, it most probably affects another parameter value but with these known systems the user is not made explicitly aware that such a change has occurred unless the change causes a value to go outside a valid min/max range of values.

During a typical prescription of a scan session, a user wants to accomplish a number of tasks, such as, reducing scan time, increasing resolution, increasing contrast, and increasing signal-to-noise ratio. Other common tasks the user may wish to accomplish during the scan prescription include increasing coverage (i.e. number of slices), entering values outside a current valid range, and providing guidance on scan parameter dependencies. Current systems are capable of assisting the user in accomplishing each of these tasks, but not easily. Further, the user must fully understand at a physics level the inter-dependencies between scan parameters and manually change these parameters in a way that accomplishes the intended result.

The present invention solves these drawbacks by demonstrating the relationship between scan parameters, notifying the user of scan parameter validity, as well as suggesting possible ways to achieve a pre-defined set of specific goals, such as reducing scan time, increasing resolution, increasing contrast, increasing signal-to-noise ration, and increasing coverage.

The present invention provides prescription guidance by notifying the user when the user changes a scan parameter value of those other scan parameters that have been automatically changed, are out of a valid range, and require the user to enter a new value. That is, if the user inputs a scan parameter value that causes another scan parameter value to be changed and the change to the other scan parameter is valid, the scan assistant will notify the user that the other scan parameter value is valid and has therefore been automatically changed. However, if the user changes a scan parameter value which causes another scan parameter to be out of the valid range, the scan assistant will notify the user that the other scan parameter is now out of a valid range and is therefore invalid. Further, if the user changes a scan parameter value, the scan assistant is also configured to notify and prompt the user to enter a new scan parameter value for another scan parameter value that is dependent upon the changed parameter value.

The present invention further provides prescription guidance by prioritizing all the scan parameters into three categories on a per scan session or experiment basis. The scan parameters are prioritized into a primary, secondary, and tertiary group. This ranking defines the relationship between parameters and provides guidance how their values may be affected based on user input. For example, change in the value of a primary parameter, such as FOV, may affect other primary parameters as well as secondary parameters, such as, resolution, and tertiary parameters, such as, timing. However, changing a secondary parameter value may affect other secondary parameters as well as tertiary parameters, but would not affect a primary parameter. Moreover, changing a tertiary parameter may only affect other tertiary parameter values. This ranking promotes the notion of driving the physics from the geometry to the timing, rather than from timing to geometry as is typically done in known systems. Because the scan assistant recognizes the parameter relationship, it may assist the user in achieving the desired timing by facilitating geometry trade-offs.

Referring to Figs. 17-21, the Scan Assistant facilitates prescribing a scan session with reduced scan time, increased resolution, increased contrast, increased signal-to-noise ratio, and increased coverage by presenting the user with these options in a series of graphical windows. The user need only select the specific task option and the Scan Assistant will then display a list of possible ways to achieve the intended result as well as displaying trade-offs associated with achieving the intended result at the expense of other limitations of the system. The displayed trade-offs or consequences may be dynamically determined based on user input or, alternatively, include a list of cann or common trade-offs associated with modifying the particular trade option.

Now referring to Fig. 17, window 338 is displayed on GUI 118 when the user selects "Scan Assistant" button 155 followed by a selection of "Scan Time" tab 340. "Scan Time" tab 340 is one of a number of tabs 342 that allows the user to complete a fixed set of tasks related to prescribing a scan session or scan experiment. The additional buttons include a "Resolution" tab 344, a "Contrast" tab 346, an "SNR" tab 348, and a "Slices" tab 350. As indicated previously, window 338 is displayed when tab 340 is selected. Window 338 displays a number of options that may be modified for the selected application that are associated with scan time. For example, the user may select reduce TR 352, reduce NEX 354, or select reduce phase-in-frequency matrix 356 to further modify scan time for the selected application. Each option further includes a checkbox 358 that the user may select to indicate to the system that an option is to be edited. The user may then input modified scan values in field 360 for each selected option. When the user inputs a scan parameter value for any option in field 360, a number of the most common consequences associated with changing that parameter value appear in field 362. This allows the user to determine, in real-time, the effects of changing a particular scan parameter value.

Window 338 further includes a number of scan parameter display fields to convey general scan parameter data to the user. These additional scan parameter values include time 364, number of slices 366, number of acquisitions 368, SNR 370, spatial resolution 372, CNR 374, DB/DT 376, Peak SAR 378, estimated SAR 380, average SAR 382, and FPS 384. A message area 386 is also provided to be used to convey messages to the user. A "Saved Series" tab 388 may be used to save modified scan parameter values.

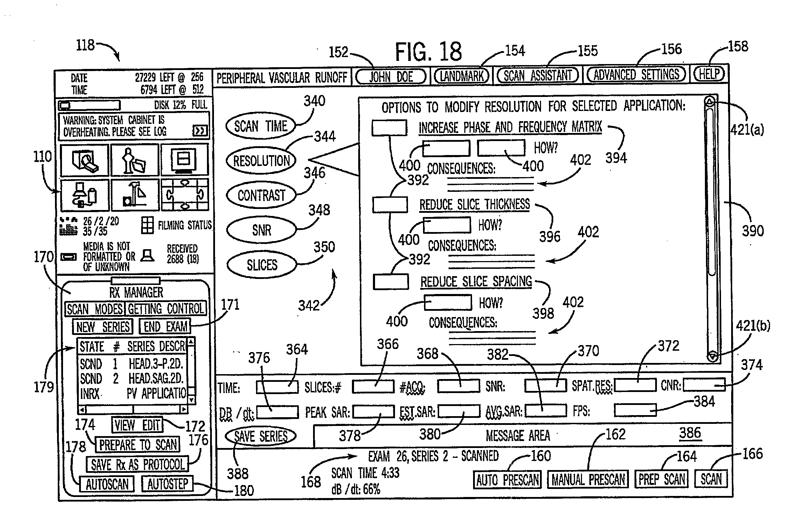

Referring to Fig. 18, when the user selects "Resolution" tab 344 window 390 is displayed that allows the user to modify the scan parameter values associated with resolution. Similar to window 338 of Fig. 17, window 390 includes a number of boxes 392 that may be selected to indicate to the system that a particular scan parameter is to be modified. In the embodiment shown in Fig. 18, the options which may be modified for the selected application related to the resolution functions include increase phase in frequency matrix 394, reduce slice thickness 396, reduce slice spacing 398, and reduce FOV (not shown). The user may input a new scan parameter value or modify an existing scan parameter for each option by entering data in fields 400 corresponding to each particular option. Inputting of a modified scan parameter value will again result in a number of consequences associated with modifying the scan parameter value to appear on window 390 in fields 402.

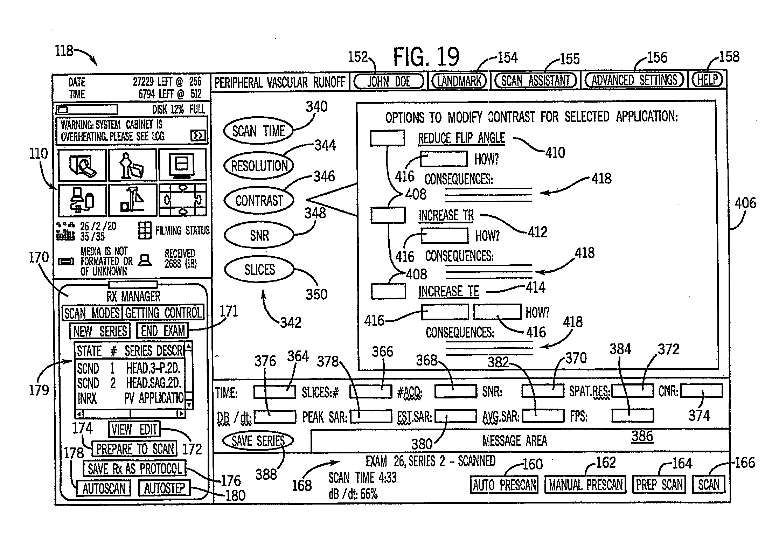

Referring to Fig. 19, selection of "Contrast" tab 346 will result in window 406 being displayed. Window 406 allows the user to modify options related to the contrast for the selected application. Boxes 408 are provided that may be "checked" to indicate that a particular option is to be modified. In this embodiment, the options include reduce flip angle 410, increase TR 412, and increase TE 414. The user may input modified data for each selected option in a corresponding field 416. When the user inputs the modified scan parameter value in field 416, the system automatically determines and displays a number of consequences associated with modifying the scan parameter value in field 418.

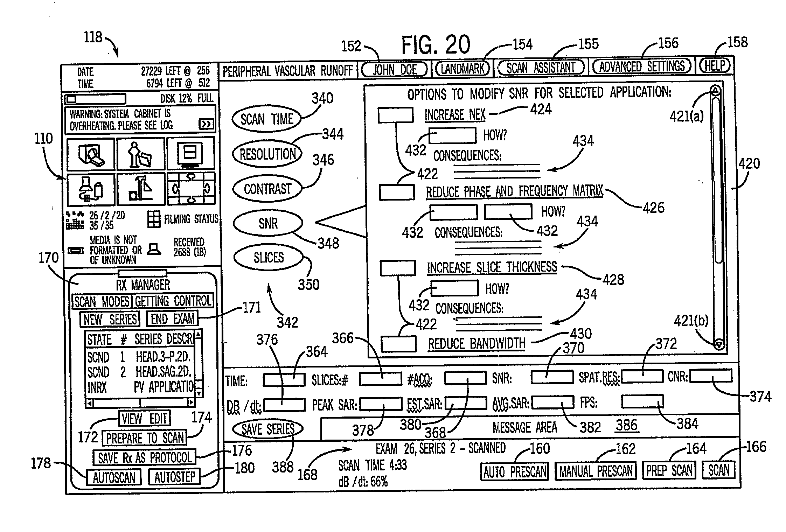

Now referring to Fig. 20, window 420 is displayed when the user selects "SNR" tab 348. Selection of "SNR" tab allows the user to modify options for the particular application related to signal-to-noise ratio. The user may indicate that a particular option is to be modified by marking box 422 corresponding to each available option. In this embodiment, the available options include increase NEX 424, reduce phase and frequency matrix 426, increase slice thickness 428, and reduce bandwidth 430. After selecting a particular option to be modified, the user may input modified scan parameter value for particular option in fields 432 which results in the system automatically determining and displaying in field 434 the consequences associated with modifying the SNR value to the value input by the user. The user may scroll window 420 using tabs 421(a) and 421(b).

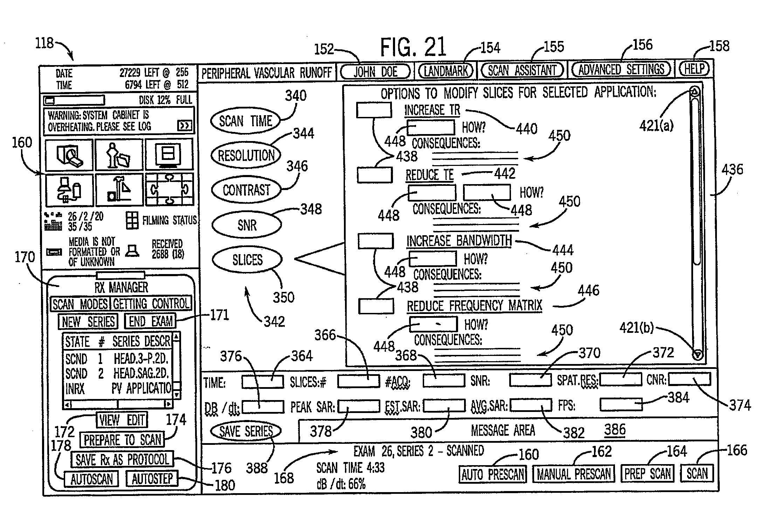

Now referring to Fig. 21, selection of "Slices" tab 350 results in window 436 being displayed to the user. Window 436 allows the user to modify options related to coverage for the selected application. The user may do so by first selecting box 438 corresponding to a particular option to be modified. In this embodiment, the modifiable options include increase TR 440, reduce TE 442, increase bandwidth 444, and reduce frequency matrix 446. After selecting an option to modify, the user may input modified scan parameter values in a corresponding field 448 for each selected option. The system will then automatically determine based on the hierarchical nature of the scan parameter values, as discussed previously, display the consequences 450 of modifying the scan parameter value as input by the user.

In another preferred embodiment, the system automatically detects modification of a parameter rather than relying on a user to first select a "check box" signaling to the system that an option is to be modified.

Once the user has modified each option desired, the user may save the modified parameters for the particular application by depressing "Save Series" tab 388. It should be noted, that the user need not view each window to save the series. That is, the user may elect to modify the options associated with scan time and contrast by viewing only those windows associated with those tabs but may elect not to modify the remaining tasks associated with a particular application. The user need not display each of the other tabs to save the series.

The present invention has been described with particular reference to a PV application implemented with an MR imaging system. However, the teachings of the present invention related to logical guidance of workflow for acquiring imaging data on a single GUI may be applicable to other medical imaging systems such as, CT, PET, X-ray, and ultra-sound.

Therefore, in accordance with one embodiment of the present invention, a method of guiding prescription of a medical imaging scan is provided. The method includes launching an imaging application and determining a plurality of scan parameters for the imaging application. The method further includes comparing a scan parameter input to a reference value and determining a state of validity of a number of remaining scan parameters. The method also includes notifying a user of the state of the validity.

In accordance with another embodiment of the present invention, a method of prescribing imaging data acquisition of subject includes determining a plurality of scan parameters specific to a scan session. The method also includes hierarchically prioritizing the plurality of scan parameters for the scan session. The method further includes re-prioritizing the plurality of scan parameters for another scan session.

In yet another embodiment, a computer program is provided and includes a set of instructions that causes a computer to display a graphical user interface (GUI) configured to assist in prescription of a medical imaging session. The instructions further cause the computer to display a window on the GUI upon receipt of a selection command wherein the window is configured to display a number modifiable scan parameters. The instructions then cause the computer to receive a command to modify a scan parameter and modify the scan parameter accordingly. The computer is then caused to determine at least one effect of modifying the scan parameter on another scan parameter and display the at least one effect on the GUI.

In a further embodiment, a medical imaging system if provided and configured to initiate an imaging application and acquire imaging data of a subject and reconstruct an image of the subject. The system includes a console configured to facilitate prescribing of a medical imaging scan. The system also includes a computer programmed to display a. plurality of tabs on the console wherein each tab corresponds to a specific task of the imaging application. The computer is further programmed to detect a user selection of one of the plurality of tabs and display on the console a plurality of options associated with the selected tab. The computer is then programmed to detect user modification of at least one of the options and display on the console an indication of at least one consequence of modifying the at least one of the plurality of options. The indication may include a system warning to the user that another scan option has an invalid value or also include an effect of modifying the at least one option.

For the sake of good order, various aspects of the invention are set out in the following clauses:-
1. A method of guiding prescription of an medical imaging scan, the method comprising:
   launching an imaging application;
   determining a plurality of scan parameters (352-356, 394-398, 410-416, 424-430, 440-446) of the imaging application;
   receiving a scan parameter input (360, 400, 416, 432 ,448);
   comparing the scan parameter input (360, 400, 416, 432, 448) to a reference value;
   determining a state of validity of a number of remaining scan parameters (352-356, 394-398, 410-416, 424-430, 440-446); and
   notifying a user of if any state of validity (362, 402, 418, 434, 450) is out of a predefined range for the scan parameter input.
2. The method of clause 1 further comprising determining and suggesting at least one technique for achieving at least one of reduced scan time (340), increased resolution (344), increased contrast (346), increased SNR (348), and increased coverage (350).
3. The method of clause 1 further comprising conveying to the user that the scan parameter input (360, 400, 416, 432, 448) is acceptable.
4. The method of clause 1 further comprising automatically updating the number of remaining parameters (352-356, 394-398, 410-416, 424-430, 440-446) in response to the scan parameter input (360, 400, 416, 432, 448).
5. The method of clause 4 wherein notifying further comprises conveying to the user that the remaining number of scan parameters (352-356, 394-398, 410-416, 424-430, 440-446) have been automatically-updated.
6. The method of clause 1 further comprising conveying to the user that the scan parameter input (360, 400, 416, 432, 448) causes at least one of the remaining scan parameters (352-356, 394-398, 410-416, 424-430, 440-446) to be invalid.
7. The method of clause 6 further comprising notifying the user of the at least one remaining invalid scan parameter (352-356, 394-398, 410-416, 424-430, 440-446).
8. The method of clause 6 further comprising prompting the user to either enter a different scan parameter input (360, 400, 416, 432, 448) or update the at least one invalid scan parameter.
9. A method of prescribing imaging data acquisition of a subject, the method comprising:
   (A) receiving a user input to initiate a scan session;
   (B) determining a plurality of scan parameters (352-356, 394-398, 410-416, 424-430, 440-446) specific to the scan session;
   (C) heirarchially prioritizing the plurality of scan parameters (352-356, 394-398, 410-416, 424-430, 440-446) for the scan session;
   (D) repeat steps (A) and (B) for a new scan session; and
   (E) re-prioritizing the plurality of scan parameters (352-356, 394-398, 410-416, 424-430, 440-446) for the new scan session.
10. The method of clause 9 wherein heirarchially prioritizing includes:
   determining a set of primary scan parameters for the scan session;
   determining a set of secondary scan parameters for the scan session; and
   determining a set of tertiary scan parameters for the scan session, wherein a change to one of the set of primary scan parameters may affect at least one of another of the set of the primary scan parameters, one of the set of secondary scan parameters and one of the set of tertiary scan parameters.
11. The method of clause 10 wherein a change to one of the set of secondary scan parameters may affect at least one of another secondary scan parameters and one of the set of the tertiary scan parameters, but may not affect one of the set of primary scan parameters.
12. The method of clause 10 wherein a change to one of the set of tertiary scan parameters may affect another of the set of tertiary scan parameters, but not affect any of the set of secondary scan parameters and any of the set of primary scan parameters.
13. The method of clause 10 further comprising organizing the set of primary scan parameters, the set of secondary scan parameters, and the set of tertiary scan parameters to drive user understanding of physics of the scan session from geometry to timing.
14. The method of clause 10 further comprising displaying the set of primary scan parameters, the set of secondary scan parameters, and the set of tertiary scan parameters on a graphical user interface (118).
15. The method of clause 14 further comprising enabling modification (358, 392, 408, 422, 438) of a displayed scan parameter.
16. The method of clause 15 further comprising displaying at least one consequence (362, 402, 418, 434, 450) of modifying a scan parameter.
17. The method of clause 14 further comprising notifying to a user that modification of a scan parameter causes another scan parameter to be invalid.
18. The method of clause 14 further comprising prompting a user to input a scan parameter value for another scan parameter as a result of the modification of the displayed scan parameter.
19. A computer readable medium having stored thereon a computer program representing a set of instructions that when executed by a computer causes the computer to:
   display a graphical user interface (GUI) (118) configured to assist prescription of a medical imaging session;
   display a window (338, 390, 406, 420, 436) on the GUI (118) upon receipt of a selection command, the window (338, 390, 406, 420, 436) configured to display a number of modifiable scan parameters (352-356, 394-398, 410-416, 424-430, 440-446);
   receive a command (360, 400, 416, 432, 450) to modify a scan parameter (352-356, 394-398, 410-416, 424-430, 440-446); modify the scan parameter (352-356, 394-398, 410-416, 424-430, 440-446);
   determine at least one effect (362, 402, 418, 434, 450) of modifying the scan parameter on another scan parameter; and
   display an indication of the at least one effect on the GUI (118).
20. The computer readable storage medium of clause 19 wherein the computer program has further instructions to determine if the modification to the scan parameter requires a change to the another scan parameter (352-356, 394-398, 410-416, 424-430, 440-446).
21. The computer readable storage medium of clause 20 wherein the computer program has further instructions to determine to what value the another scan parameter (352-356, 394-398, 410-416, 424-430, 440-446) should be changed and determine if that value is valid.
22. The computer readable storage medium of clause 21 wherein the computer program has further instructions to automatically change the another scan parameter (352-356, 394-398, 410-416, 424-430, 440-446) to the valid value.
23. The computer readable storage medium of clause 20 wherein the computer program has further instructions to display that the another scan parameter (352-356, 394-398, 410-416, 424-430, 440-446) has an invalid value on the GUI (118).
24. The computer readable storage medium of clause 20 wherein the computer program has further instructions to prompt a user to modify the another scan parameter (352-356, 394-398, 410-416, 424-430, 440-446).
25. A medical imaging system (10) configured to initiate an imaging application and acquire imaging data of a subject and reconstruct a diagnostic image of the subject, the system having:
   a console (16) configured to facilitate prescribing of a medical imaging scan; and
   a computer (20a) programmed to:
      display a plurality of tabs (342) on the console (16) wherein each tab (340, 344, 346, 348, 350) corresponds to a specific task of the imaging application;
      detect a user selection (338, 390, 406, 420, 436) of one of the plurality of tabs (342);
      display on the console (16) a plurality of options (352-356, 394-398, 410-416, 424-430, 440-446) associated with the selected tab (340, 344-350);
      detect user modification (360, 400, 416, 432, 450) of at least one of the plurality of options (352-356, 394-398, 410-416, 424-430, 440-446); and
      display on the console (16) if there is any consequence (362, 402, 418, 434, 450) of modifying the at least one of the plurality of options on another option.
26. The medical imaging system of clause 25 wherein the computer is further programmed to determine if the user modification (360, 400, 416, 432, 450) causes a change to a scan parameter (352-356, 394-398, 410-416, 424-430, 440-446), and if so, determine if another scan parameter (352-356, 394-398, 410-416, 424-430, 440-446) needs modification.
27. The medical imaging system of clause 26 wherein the computer is further programmed to determine a value by which to change the another scan parameter (352-356, 394-398, 410-416, 424-430, 440-446).
28. The medical imaging system of clause 27 wherein the computer is further programmed to determine if the value is valid, and if so, automatically change the another scan parameter (352-356, 394-398, 410-416, 424-430, 440-446) to the value, and if not, display a message on the console that the another scan parameter (352-356, 394-398, 410-416, 424-430, 440-446) has an invalid value.
29. The medical imaging system of clause 27 wherein the computer is further programmed to prompt a user to enter a new value for the another scan parameter (352-356, 394-398, 410-416, 424-430, 440-446).
30. The medical imaging system of clause 25 wherein the plurality of options (352-356, 394-398, 410-416, 424-430, 440-446) are enclosed in a window (338, 390, 406, 420, 436), wherein the window (338, 390, 406, 420, 436) is displayed to the right of the plurality of tabs (342) and wherein the plurality of tabs (342) are vertically oriented.
31. The medical imaging system of clause 30 wherein the computer is further programmed to display the plurality of tabs (342) and the plurality of options (352-356, 394-398, 410-416, 424-430, 440-446) enclosed in the window to facilitate a logical top-bottom and left-right workflow for prescribing a medical imaging scan session.

## Claims

1. A method of guiding prescription of an medical imaging scan, the method comprising:
launching an imaging application;
determining a plurality of scan parameters (352-356, 394-398, 410-416, 424-430, 440-446) of the imaging application;
receiving a scan parameter input (360, 400, 416, 432 ,448);
comparing the scan parameter input (360, 400, 416, 432, 448) to a reference value;
determining a state of validity of a number of remaining scan parameters (352-356, 394-398, 410-416, 424-430, 440-446); and
notifying a user of if any state of validity (362, 402, 418, 434, 450) is out of a predefined range for the scan parameter input.

2. The method of claim 1 further comprising determining and suggesting at least one technique for achieving at least one of reduced scan time (340), increased resolution (344), increased contrast (346), increased SNR (348), and increased coverage (350).

3. The method of claim 1 or 2f urther comprising conveying to the user that the scan parameter input (360, 400, 416, 432, 448) is acceptable.

4. A method of prescribing imaging data acquisition of a subject, the method comprising:
(A) receiving a user input to initiate a scan session;
(B) determining a plurality of scan parameters (352-356, 394-398, 410-416, 424-430, 440-446) specific to the scan session;
(C) heirarchially prioritizing the plurality of scan parameters (352-356, 394-398, 410-416, 424-430, 440-446) for the scan session;
(D) repeat steps (A) and (B) for a new scan session; and
(E) re-prioritizing the plurality of scan parameters (352-356, 394-398, 410-416, 424-430, 440-446) for the new scan session.

5. The method of claim 4 wherein heirarchially prioritizing includes:
determining a set of primary scan parameters for the scan session;
determining a set of secondary scan parameters for the scan session; and
determining a set of tertiary scan parameters for the scan session, wherein a change to one of the set of primary scan parameters may affect at least one of another of the set of the primary scan parameters, one of the set of secondary scan parameters and one of the set of tertiary scan parameters.

6. The method of claim 5 wherein a change to one of the set of secondary scan parameters may affect at least one of another secondary scan parameters and one of the set of the tertiary scan parameters, but may not affect one of the set of primary scan parameters.

7. A computer readable medium having stored thereon a computer program representing a set of instructions that when executed by a computer causes the computer to:
display a graphical user interface (GUI) (118) configured to assist prescription of a medical imaging session;
display a window (338, 390, 406, 420, 436) on the GUI (118) upon receipt of a selection command, the window (338, 390, 406, 420, 436) configured to display a number of modifiable scan parameters (352-356, 394-398, 410-416, 424-430, 440-446);
receive a command (360, 400, 416, 432, 450) to modify a scan parameter (352-356, 394-398, 410-416, 424-430, 440-446);
modify the scan parameter (352-356, 394-398, 410-416, 424-430, 440-446);
determine at least one effect (362, 402, 418, 434, 450) of modifying the scan parameter on another scan parameter; and
display an indication of the at least one effect on the GUI (118).

8. The computer readable storage medium of claim 7 wherein the computer program has further instructions to determine if the modification to the scan parameter requires a change to the another scan parameter (352-356, 394-398, 410-416, 424-430, 440-446).

9. A medical imaging system (10) configured to initiate an imaging application and acquire imaging data of a subject and reconstruct a diagnostic image of the subject, the system having:
a console (16) configured to facilitate prescribing of a medical imaging scan; and
a computer (20a) programmed to:
display a plurality of tabs (342) on the console (16) wherein each tab (340, 344, 346, 348, 350) corresponds to a specific task of the imaging application;
detect a user selection (338, 390, 406, 420, 436) of one of the plurality of tabs (342);
display on the console (16) a plurality of options (352-356, 394-398, 410-416, 424-430, 440-446) associated with the selected tab (340, 344-350) ;
detect user modification (360, 400, 416, 432, 450) of at least one of the plurality of options (352-356, 394-398, 410-416, 424-430, 440-446); and
display on the console (16) if there is any consequence (362, 402, 418, 434, 450) of modifying the at least one of the plurality of options on another option.

10. The medical imaging system of claim 9 wherein the computer is further programmed to determine if the user modification (360, 400, 416, 432, 450) causes a change to a scan parameter (352-356, 394-398, 410-416, 424-430, 440-446), and if so, determine if another scan parameter (352-356, 394-398, 410-416, 424-430, 440-446) needs modification.
